# EUROPEAN PATENT SPECIFICATION

(11) **EP 4 370 036 B1**
(45) Date of publication and mention of the grant of the patent: **04.12.2024**
(21) Application number: 22748045.6
(22) Date of filing: 13.07.2022
(51) Int. Cl.: A61B 8/06, A61B 8/08, A61B 8/00

(54) **SYSTEMS AND METHODS FOR SPECTRAL PULSE WAVE DOPPLER ULTRASOUND MEASUREMENTS**
SYSTEME UND VERFAHREN FÜR DOPPLER-ULTRASCHALLMESSUNGEN
SYSTÈMES ET PROCÉDÉS DE MESURES D'ULTRASONS DOPPLER À ONDES PULSÉES SPECTRALES

(30) Priority: 15.07.2021 EP 21290047
(43) Date of publication of application: 22.05.2024
(73) Proprietor: Koninklijke Philips N.V., 5656 AG Eindhoven (NL)
(72) Inventor: CLARK, David Wesley, 5656 AG Eindhoven (NL); BADESCU, Emilia, 5656 AG Eindhoven (NL); BONNEFOUS, Odile, 5656 AG Eindhoven (NL); RADHAKRISHNAN, Kirthi, 5656 AG Eindhoven (NL)
(74) Representative: Philips Intellectual Property & Standards
(86) International application number: PCT/EP2022/069577
(87) International publication number: WO 2023/285517

(56) References cited:
- US-A- 5 899 864
- US-A1- 2016 151 038
- "2-D tracking doppler: a new method to limit spectral broadening in pulsed wave doppler", IEEE TRANSACTIONS ON ULTRASONICS, FERROELECTRICS, AND FREQUENCY CONTROL, IEEE, USA, vol. 60, no. 9, 1 September 2013 (2013-09-01), XP011525471, ISSN: 0885-3010, [retrieved on 20130827], DOI: 10.1109/TUFFC.2013.2774
- AVDAL JORGEN ET AL: "Combined 2-D Vector Velocity Imaging and Tracking Doppler for Improved Vascular Blood Velocity Quantification", IEEE TRANSACTIONS ON ULTRASONICS, FERROELECTRICS, AND FREQUENCY CONTROL, IEEE, USA, vol. 64, no. 12, 1 December 2017 (2017-12-01), pages 1795 - 1804, XP011673746, ISSN: 0885-3010, [retrieved on 20171201], DOI: 10.1109/TUFFC.2017.2757600
- FREDRIKSEN TONJE ET AL: "Investigations of spectral resolution and angle dependency in a 2-D tracking doppler method", IEEE TRANSACTIONS ON ULTRASONICS, FERROELECTRICS, AND FREQUENCY CONTROL, IEEE, USA, vol. 61, no. 7, 1 July 2014 (2014-07-01), pages 1161 - 1170, XP011552210, ISSN: 0885-3010, [retrieved on 20140617], DOI: 10.1109/TUFFC.2014.3015
- FIORENTINI STEFANO ET AL: "3D tracking Doppler for improved quantitative blood flow assessment of coronary arteries", 2017 IEEE INTERNATIONAL ULTRASONICS SYMPOSIUM (IUS), IEEE, 6 September 2017 (2017-09-06), pages 1 - 4, XP033245595, DOI: 10.1109/ULTSYM.2017.8092469

## Description

### FIELD OF THE INVENTION

This application relates to spectral Doppler imaging of blood vessels. More specifically, this application relates to improving spectral Doppler measurements by tracking the blood vessels over time.

### BACKGROUND OF THE INVENTION

Fredriksen et al. (2013) "2-D Tracking Doppler: A New Method to Limit Spectral Broadening in Pulsed Wave Doppler" discloses a method to overcome velocity overestimations when the angle between the flow direction and the ultrasound beam is large.

Avdal et al. (2017) "Combined 2-D Vector Velocity Imaging and Tracking Doppler for Improved Vascular Blood Velocity Quantification" discloses combining two ultrasound velocity estimation methods to reduce the bias and variability in maximum velocity measurements.

US 2016/0151038 (Govinahallisathyanarayana et al. 2013) discloses a method for aligning spatially different subvolumes of ultrasonic data of a blood vessel.

Ultrasound Doppler imaging is typically used in a qualitative manner to determine the presence or absence of flow. Doppler imaging may also be used to visualize vessels through anatomy as an alternative to more invasive procedures, such as angiograms. For example, Doppler imaging may be used to image coronary arteries in children to diagnose and/or grade conditions (e.g., congenital defects, Kawasaki Disease). Imaging of coronary arteries in adults is also becoming more common, particularly in Europe and Asia, to diagnose and/or grade coronary artery disease. In this application, Doppler may be used in a quantified manner to calculate certain parameters related to flow. For example, low values of coronary flow reserve (CFR), which is a ratio between maximal and resting coronary blood flow, has been found to be correlated to coronary artery disease.

However, acquiring reliable quantitative measurements for velocity from Doppler data may be challenging. For example, Doppler data usually requires angle correction to obtain accurate velocities. Typically, a user must manually provide an indication of the angle, thus, the correction is user-dependent. Coronary arteries are challenging to image with color flow imaging, and even more challenging to quantify with spectral pulse-wave (PW) Doppler, due to the significant tissue and vessel motion. This may make it difficult to maintain the vessel within the imaging plane and the PW sample gate. Furthermore, coronary arteries are relatively small, particularly in pediatric cases. Thus, it may be difficult to find transducer placement "windows" that are optimally aligned with the vessel. Furthermore, flow signals from the coronary arteries may be difficult to discern from the fast, strong blood flow nearby (e.g., within the chambers of the heart, aorta). Accordingly, improved techniques for quantitative Doppler imaging of blood vessels may be desirable.

### SUMMARY OF THE INVENTION

Apparatuses, systems, and methods disclosed herein may form an RF or IQ anatomical M-line ("fast time") along an axis of a target vessel and spatially register the M-line to the axis of the vessel over time as the vessel moves. "Slow time" spectral Doppler may be performed with the M-line samples, generating a registered, angle-corrected Doppler signal from the target vessel. Techniques are described for generating Doppler audio and spectral estimates.

In some examples, an imaging system automatically finds and tracks a coronary artery in a three-dimensional (3D) cluster of receive beams and depth around a nominal pulsewave cursor location. The artery location may be considered fixed within individual ones of overlapping spans of time data used for spectral processing, which may eliminate spectral artifacts from vessel motion tracking. Data from along the artery within a region of interest (ROI) may be coherently integrated, which may improve sensitivity and/or reduce spectral broadening. In some examples, the beam-to-flow angle may be automatically compensated. In some examples, a live, enlarged 3D rendered graphic or color flow image of the ROI may be provided on a display, which may help the user maintain transducer position while the spectrogram is displaying without interruption.

The invention is defined by the independent claims. The dependent claims define advantageous embodiments.

### BRIEF DESCRIPTION OF THE DRAWINGS

Fig. 1 is a block diagram of an ultrasound imaging system arranged in accordance with principles of the present disclosure.
Fig. 2 illustrates examples of suitable azimuth-elevation multiline receive beam patterns according to principles of the present disclosure.
Fig. 3 illustrates overlapping time segments according to principles of the present disclosure.
Fig. 4 is an image of a contour fitted to a vessel according to principles of the present disclosure.
Fig. 5 is a flow chart of a method according to principles of the present disclosure.
Fig. 6 is an example of an output of a display of an ultrasound imaging system in accordance with principles of the present disclosure.
Fig. 7 is a block diagram illustrating an example processor in accordance with principles of the present disclosure.

### DETAILED DESCRIPTION OF EMBODIMENTS

The following description of certain exemplary examples is merely exemplary in nature and is in no way intended to limit the invention or its applications or uses. In the following detailed description of examples of the present systems and methods, reference is made to the accompanying drawings which form a part hereof, and in which are shown by way of illustration specific examples in which the described apparatuses, systems and methods may be practiced. These examples are described in sufficient detail to enable those skilled in the art to practice the presently disclosed systems and methods, and it is to be understood that other examples may be utilized, and that structural and logical changes may be made without departing from the scope of the present disclosure. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present invention is defined only by the appended claims.

Spectral Doppler refers to spectral analysis of Doppler data. A user may select a region of interest (ROI) within a B-mode image for performing Doppler imaging. In some applications, the user may select the ROI for color flow Doppler imaging and then select a sub-region within the ROI for acquiring spectral Doppler data. In some applications, the user may select the ROI within M-mode images for performing Doppler imaging.

Several transmit-receive events are performed in the ROI or sub-region to acquire spectral Doppler data. The acquired spectral Doppler data may be analyzed, for example, by performing a Fast Fourier Transform (FFT), to provide a spectrum of frequencies present in the spectral Doppler data. The frequencies may be converted to velocities, which may correspond to velocities of fluid flow in the ROI or sub-region, for example, velocities of blood in a blood vessel. The velocity in the ROI may be plotted over time as a spectrogram. The velocities may be analyzed qualitatively or quantitatively, such as for calculating CFR, for diagnosing and/or grading a medical condition. While operating in the spectral Doppler mode, the user must maintain a position of the transducer array. With traditional uninterrupted spectral PW Doppler, this is challenging for coronary arteries because there is no visual feedback besides the spectrum, and the artery is often difficult to find with color flow even before entering the spectral mode.

Researchers have shown that velocity estimates for spectral PW Doppler may be improved by integrating data along a stationary straight line, where the straight line approximates a vessel axis through a small 3D acquisition volume. *See* S. Fiorentini, L.M. Saxhaug, T. Bjastad, E. Holte, H. Torp, J. Avdal; "Maximum velocity estimation in coronary arteries using 3D tracking Doppler"; IEEE Trans UFFC 65/7, pp 1102-1110, 4/16/2018. However, these techniques at most track scatters (e.g., blood cells) within the bloodstream. Thus, while the existing techniques may reduce spectral broadening, they do not provide an optimal signal-to-noise ratio, nor do they compensate for the motion of the blood vessel over time (e.g., due to probe or cardiac tissue movement) or curves in the blood vessel. As disclosed herein, systems and methods may compensate for the motion of the blood vessel as well as take into account data along the vessel, even if the vessel is curved. Thus, the signal-to-noise ratio may be optimized, and spectral spread may be minimized.

According to examples of the present disclosures, a coronary artery may automatically be identified and tracked within a small 3D cluster of receive beams and depth around a nominal PW cursor location, also referred to as a region of interest (ROI), which may be selected automatically or by a user. The artery location may be considered fixed within individual ones of overlapping spans of time data (e.g., time periods, time segments) used for spectral processing, to eliminate spectral artifacts from vessel motion tracking. Ultrasound data from along the artery within the ROI may be coherently integrated. Coherently integrating means summing complex data (data including real and imaginary parts), with a time delay and/or a phase shift (if necessary) corresponding to a relative axial position of the data. In some applications, this may provide improved sensitivity and reduced spectral broadening. A FFT may be performed on the coherently integrated data to provide a spectrogram of the blood flow in the coronary artery. The beam-to-flow angle may be compensated for automatically. Optionally, in some examples, a live, enlarged 3D rendered graphic or color flow image of the ROI may be provided on a display. In some applications, the graphic of the ROI may help the user maintain transducer position to allow the spectrogram to be provided without interruption. Optionally, Doppler audio output also may be produced from the processing.

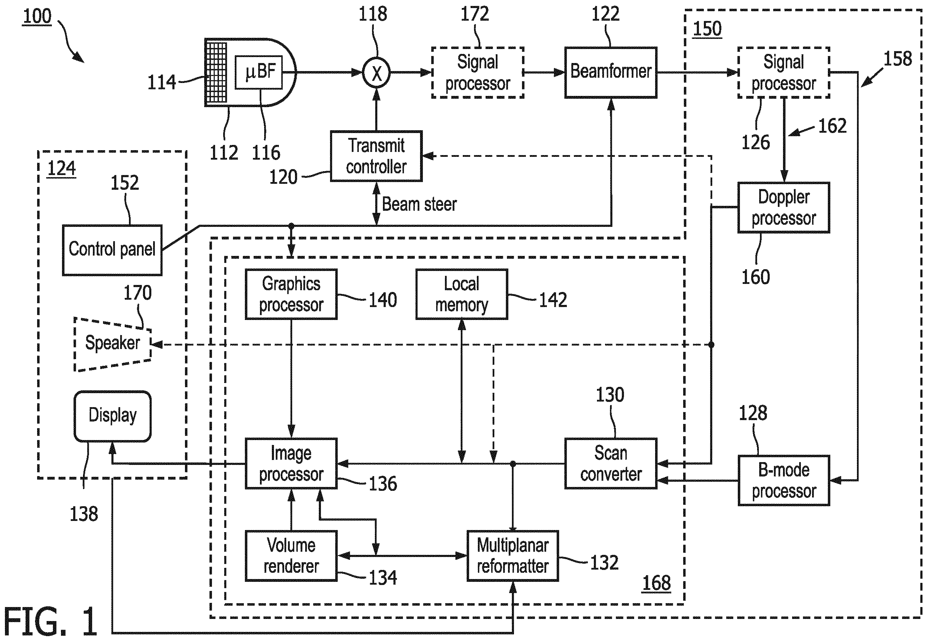

Fig. 1 shows a block diagram of an ultrasound imaging system 100 constructed in accordance with the principles of the present disclosure. An ultrasound imaging system 100 according to the present disclosure may include a transducer array 114, which may be included in an ultrasound probe 112, for example an external probe or an internal probe. The transducer array 114 is configured to transmit ultrasound signals (e.g., beams, waves) and receive echoes (e.g., received ultrasound signals) responsive to the transmitted ultrasound signals. A variety of transducer arrays may be used, e.g., linear arrays, curved arrays, or phased arrays. The transducer array 114, for example, can include a two-dimensional array (as shown) of transducer elements capable of scanning in both elevation and azimuth dimensions for 2D and/or 3D imaging. As is generally known, the axial direction is the direction normal to the face of the array (in the case of a phased or linear array the axial direction may be steered away from normal to the array face and, in the case of a curved array the axial directions fan out), the azimuthal direction is defined generally by the longitudinal dimension of the array (normal to the axial), and the elevation direction is transverse to the azimuthal direction.

In some examples, the transducer array 114 may be coupled to a microbeamformer 116, which may be located in the ultrasound probe 112, and which may control the transmission and reception of signals by the transducer elements in the array 114. In some examples, the microbeamformer 116 may control the transmission and reception of signals by active elements in the array 114 (e.g., an active subset of elements of the array that define the active aperture at any given time).

In some examples, the microbeamformer 116 may be coupled, e.g., by a probe cable or wirelessly, to a transmit/receive (T/R) switch 118, which switches between transmission and reception and protects the main beamformer 122 from high energy transmit signals. In some examples, for example in portable ultrasound systems, the T/R switch 118 and other elements in the system can be included in the ultrasound probe 112 rather than in the ultrasound system base, which may house the image processing electronics. An ultrasound system base typically includes software and hardware components including circuitry for signal processing and image data generation as well as executable instructions for providing a user interface.

The transmission of ultrasonic signals from the transducer array 114 under control of the microbeamformer 116 is directed by the transmit controller 120, which may be coupled to the T/R switch 118 and a main beamformer 122. The transmit controller 120 may control the direction in which beams are steered. Beams may be steered straight ahead from (orthogonal to) the transducer array 114, or at different angles for a wider field of view. The transmit controller 120 may also be coupled to a user interface 124 and receive input from the user's operation of a user control. The user interface 124 may include one or more input devices such as a control panel 152, which may include one or more mechanical controls (e.g., buttons, encoders, etc.), touch sensitive controls (e.g., a trackpad, a touchscreen, or the like), and/or other known input devices.

In some examples, the partially beamformed signals produced by the microbeamformer 116 may be coupled to a main beamformer 122 where partially beamformed signals from individual patches of transducer elements may be combined into a fully beamformed signal. In some examples, microbeamformer 116 is omitted, and the transducer array 114 is under the control of the beamformer 122 and beamformer 122 performs all beamforming of signals. In examples with and without the microbeamformer 116, the beamformed signals of beamformer 122 are coupled to processing circuitry 150, which may include one or more processors (e.g., a signal processor 126, a B-mode processor 128, a Doppler processor 160, and one or more image generation and processing components 168) configured to produce an ultrasound image from the beamformed signals (i.e., beamformed RF data).

The signal processor 126 may process the received beamformed RF data in various ways, such as bandpass filtering, decimation, I (in-phase) and Q (quadrature-phase) complex component separation, and harmonic signal separation. The signal processor 126 may also perform additional signal enhancement such as speckle reduction, signal compounding, and electronic noise elimination. The processed signals (also referred to as I and Q components or IQ signals) may be coupled to additional downstream signal processing circuits for image generation. The IQ signals may be coupled to a plurality of signal paths within the system, each of which may be associated with a specific arrangement of signal processing components suitable for generating different types of image data (e.g., B-mode image data, contrast image data, Doppler image data). For example, the system 100 may include a B-mode signal path 158 which couples the signals from the signal processor 126 to a B-mode processor 128 for producing B-mode image data. The B-mode processor 128 can employ amplitude detection for the imaging of organ structures the body.

Although the signal processor 126 is described as after the beamformer 122, in other examples, the signal processor 126 may receive and process signals from the transducer array 114 prior to beamforming and provide the processed signals to the beamformer 122. In some examples, the system 100 may include two signal processors 126 and 172, where signal processor 172 receives and process signals received from the transducer array 114 and signal processor 126 receives and process signals received from the beamformer 122.

In some examples, the system may include a Doppler signal path 162 which couples the output from the signal processor 126 to a Doppler processor 160. The Doppler processor 160 may be configured to estimate the Doppler shift and generate Doppler image data. The Doppler image data may include color data which is then overlaid with B-mode (e.g., grayscale) image data for display. The Doppler processor 160 may be configured to filter out unwanted signals (e.g., noise or clutter associated with stationary or slow moving tissue), for example using a wall filter. The Doppler processor 160 may be further configured to estimate velocity and power in accordance with known techniques. For example, the Doppler processor may include a Doppler estimator such as an auto-correlator, in which velocity (Doppler frequency, color Doppler) estimation is based on the argument of the lag-one (R1) autocorrelation function and Doppler power estimation is based on the magnitude of the lag-zero (R0) autocorrelation function. Motion can also be estimated by known phase-domain (for example, parametric frequency estimators such as MUSIC, ESPRIT, etc.) or time-domain (for example, cross-correlation) signal processing techniques. Other estimators related to the temporal or spatial distributions of velocity such as estimators of acceleration or temporal and/or spatial velocity derivatives can be used instead of or in addition to velocity estimators. In some examples, such as for spectral Doppler, a fast Fourier transform (FFT) may be performed on the Doppler data to provide a spectrum of frequencies included in the Doppler data within one or more time periods. The frequencies may be converted into velocities. In some examples, the velocity and power estimates may undergo further threshold detection to further reduce noise, as well as segmentation and post-processing such as filling and smoothing.

In some examples, velocity and power estimates may then be mapped to a desired range of display colors and/or intensities in accordance with a color and/or intensity map. The color and/or intensity data, also referred to as Doppler image data, may then be coupled to the scan converter 130, where the Doppler image data may be converted to the desired image format and overlaid on the B-mode image of the tissue structure to form a color Doppler or a power Doppler image. For example, Doppler image data may be overlaid on a B-mode image of the tissue structure.

In some examples, the velocity data derived from the Doppler image data may be plotted over time to provide a spectrogram. The spectrogram may be displayed concurrently with the B-mode image, color Doppler image data, power Doppler image data, or combinations thereof. In other examples, only the spectrogram may be displayed. In some examples, the frequency data used to generate the velocity data may be used to generate audible sounds for output by a speaker 170. Low frequency sounds are associated with slow (low) velocity flows and high frequency sounds are associated with fast (high) velocity flows.

The signals produced by the B-mode processor 128 and/or Doppler processor 160 may be coupled to a scan converter 130 and/or a multiplanar reformatter 132. The scan converter 130 may be configured to arrange the echo signals from the spatial relationship in which they were received to a desired image format. For instance, the scan converter 130 may arrange the echo signal into a two-dimensional (2D) sector-shaped format, or a pyramidal or otherwise shaped three-dimensional (3D) format. The multiplanar reformatter 132 can convert echoes which are received from points in a common plane in a volumetric region of the body into an ultrasonic image (e.g., a B-mode image) of that plane, for example as described in U.S. Pat. No. 6,443,896 (Detmer). The scan converter 130 and multiplanar reformatter 132 may be implemented as one or more processors in some examples.

A volume renderer 134 may generate an image (also referred to as a projection, render, or rendering) of the 3D dataset as viewed from a given reference point, e.g., as described in U.S. Pat. No. 6,530,885 (Entrekin et al.). The volume renderer 134 may be implemented as one or more processors in some examples. The volume renderer 134 may generate a render, such as a positive render or a negative render, by any known or future known technique such as surface rendering and maximum intensity rendering. Although shown in Fig. 1 as receiving data from the multiplanar reformatter 132, in some examples, the volume renderer 134 may receive data from the scan converter 130.

Output (e.g., B-mode images, Doppler images) from the scan converter 130, the multiplanar reformatter 132, and/or the volume renderer 134 may be coupled to an image processor 136 for further enhancement, buffering and temporary storage before being displayed on an image display 138. Optionally, in some examples, some Doppler data, such as spectral Doppler data that may include frequencies and/or velocities plotted over time, may be provided directly from the Doppler processor 160 to the image processor 136 for generating a spectrogram or other desired data output formats. A graphics processor 140 may generate graphic overlays for display with images, such as the accumulation image generated by the image processor 136. These graphic overlays can contain, e.g., standard identifying information such as patient name, date and time of the image, imaging parameters, and the like. For these purposes the graphics processor may be configured to receive input from the user interface 124, such as a typed patient name or other annotations. The user interface 124 can also be coupled to the multiplanar reformatter 132 for selection and control of a display of multiple multiplanar reformatted (MPR) images.

The system 100 may include local memory 142. Local memory 142 may be implemented as any suitable non-transitory computer readable medium or media (e.g., flash drive, disk drive, dynamic random-access memory). Local memory 142 may store data generated by the system 100 including B-mode images, Doppler images, instructions capable of being executed by one or more of the processors included in the system 100 (e.g., Doppler processor 160, image processor 136), inputs provided by a user via the user interface 124, or any other information necessary for the operation of the system 100. As mentioned previously, system 100 includes user interface 124. User interface 124 may include display 138 and control panel 152. The display 138 may include a display device implemented using a variety of known display technologies, such as LCD, LED, OLED, or plasma display technology. In some examples, display 138 may comprise multiple displays. The control panel 152 may be configured to receive user inputs (e.g., imaging mode, selection of ROI in image). The control panel 152 may include one or more hard controls (e.g., buttons, knobs, dials, encoders, mouse, trackball or others). In some examples, the control panel 152 may additionally or alternatively include soft controls (e.g., GUI control elements or simply, GUI controls) provided on a touch sensitive display. In some examples, display 138 may be a touch sensitive display that includes one or more soft controls of the control panel 152. Optionally, in some examples, the user interface 124 may include a speaker 170 for providing audio signals to the user. For example, speaker 170 may provide audio signals received from the Doppler processor 160, which may be associated with Doppler frequencies detected by the Doppler processor 160.

In some examples, various components shown in Fig. 1 may be combined. For instance, image processor 136 and graphics processor 140 may be implemented as a single processor. In another example, the scan converter 130 and multiplanar reformatter 132 may be implemented as a single processor. In some examples, various components shown in Fig. 1 may be implemented as separate components. For example, Doppler processor 160 may be implemented as multiple processors. In some examples, the multiple processors may perform different tasks (e.g., spectral Doppler, power Doppler, etc.). In another example, local memory 142 may include multiple memories which may be the same or different memory types (e.g., flash, DRAM).

In some examples, one or more of the various processors shown in Fig. 1 may be implemented by general purpose processors and/or microprocessors configured to perform the specified tasks. For example, the processors may be configured by instructions stored in a non-transitory computer readable memory (e.g., local memory 142) which are executed by the processors to perform the specified tasks. In some examples, one or more of the various processors may be implemented as application specific circuits (ASICs). In some examples, one or more of the various processors (e.g., image processor 136) may be implemented with one or more graphical processing units (GPU).

According to examples of the present disclosure, a user may indicate a ROI and Doppler data may then be acquired within the ROI. Color flow Doppler, power Doppler data, other Doppler data, or combinations thereof, may be used to detect a blood vessel within the ROI. A 1D contour (e.g., curve) along a center axis of the blood vessel may be plotted through the ROI. Spectral PW Doppler data from along the contour may be acquired and used to generate velocity estimates. Thus, spectral PW Doppler data may be acquired from multiple locations within the volume rather than from a single location. Detection of the vessel, calculation of the contour, and integration of the spectral PW Doppler data may be repeated for multiple time segments. By using power Doppler to detect the vessel, the vessel may be effectively tracked over time. Furthermore, calculating a contour along a center of the portion of the vessel within the ROI and integrating along the contour allows the spectral PW Doppler data to account for curves within the vasculature.

In some examples according to the present disclosure, a user may indicate a ROI and M-mode (or motion mode) data may then be acquired within the ROI. M-mode data, similarily to Doppler data, or combinations thereof, may be used to detect a blood vessel within the ROI. By repeatedly using M-mode ultrasound, multiline received M-lines, can be processed similarily as PW Doppler to generate image data, such as Doppler images, color Doppler images. That as both PW Doppler and M-mode transmit a short acoustic burst periodically in the same direction and receive echoes from that direction, so from an ultrasound acoustic standpoint PW Doppler and M-mode essentially generate equivalent ultrasound data, which may then be processed to display images of echo intensity (e.g., B-mode), images or overlays of blood flow average velocity or power (like color velocity or power), or Doppler spectrograms.

In some examples, a graphic of the calculated contour of the blood vessel may be generated (e.g., by image processor 136 and/or graphics processor 140) and provided on display 138. This may assist the user in visualizing the location of the vessel within the ROI and adjust the probe 112 to maintain the vessel within the ROI. Optionally, the calculated contour of the blood vessel may be provided to the transmit controller 120, which may automatically adjust the ROI and/or steering of the transducer array 114 to maintain the detected vessel within the ROI. Thus, by utilizing Doppler data to detect the blood vessel and integrating the PW Doppler data along a curve of the blood vessel, the blood vessel may be tracked over time and improved velocity estimates from the spectral PW Doppler data may be achieved. The techniques will now be described in greater detail.

In some examples, a user may indicate a ROI including a vessel, such as a coronary vessel via user interface 124. For example, the user may select the ROI from a B-mode image and/or color flow Doppler image provided on display 138. In some examples, the ROI may be 3D (e.g., a volume). Doppler data may then be acquired within the ROI by transmitting ultrasound waves and receiving ultrasound signals with the transducer array 114. The transducer array 114 may acquire spectral PW Doppler data from the ROI at a pulse repetition frequency (PRF) using multiline receive beamforming. In these examples, transducer array 114 may be a matrix transducer. In examples where the ROI is 2D, transducer array 114 may be a 1D transducer.

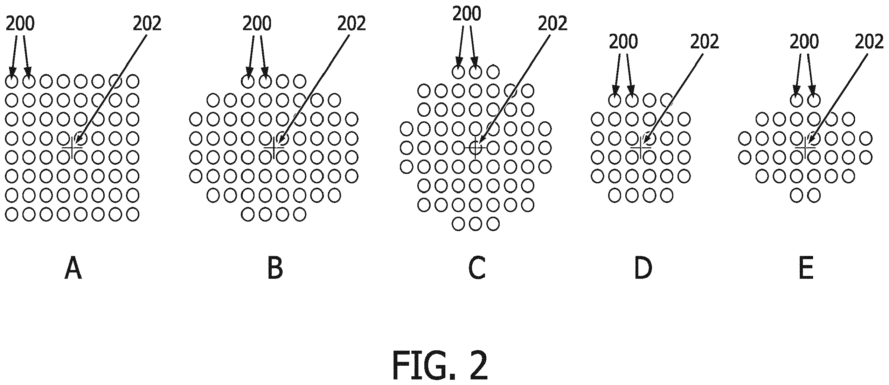

Fig. 2 illustrates examples of suitable azimuth-elevation multiline receive beam patterns according to principles of the present disclosure. In the examples A-E, the receive beams received by the transducer array 114 are indicated by the circles 200 and a center of the transmit beam is indicated by a cross 202. In some examples, the aspect ratio may be changed to suit the aperture geometry. In some examples, the transmit beam transmitted by the transducer array 114 may be relatively broad at the ROI depth to cover the cluster of receive beams.

Signals resulting from the received echoes may be a complex mix (e.g., real and imaginary parts) that is generated from baseband conversion and/or filtering (e.g., quadrature filtering) in some examples. In some examples, the data is not integrated over a single temporal range gate as in traditional spectral PW Doppler. However, complex RF bandpass filtering and/or baseband conversion may be performed. In some examples, complex RF bandpass filtering and/or baseband conversion may have somewhat similar effects to integrating over a single range (e.g., temporal range) gate, although producing many data samples per transmit instead of only one. The complex data may be clutter filtered in the slow-time dimension (e.g., PRF) to remove tissue echoes. The filtering could be fixed-coefficient infinite impulse response (IIR) or finite impulse response (FIR) for each spatial sample, or other more sophisticated clutter/flow separation techniques. In some examples, the filtering may be performed by the signal processor 126 and/or 172. In some examples, the signals may be beamformed by main beamformer 122 prior to filtering or other processing by the signal processor 126. In some examples, only data from an axial ROI (which may correspond to the ROI selected by the user in some examples) may be processed by the signal processor 126. In some examples, the beamformed and filtered data may be provided to the Doppler processor 160 to derive various types of Doppler data from the filtered ultrasound signals. In other examples, the filtering and/or other processing may be performed by the Doppler processor 160.

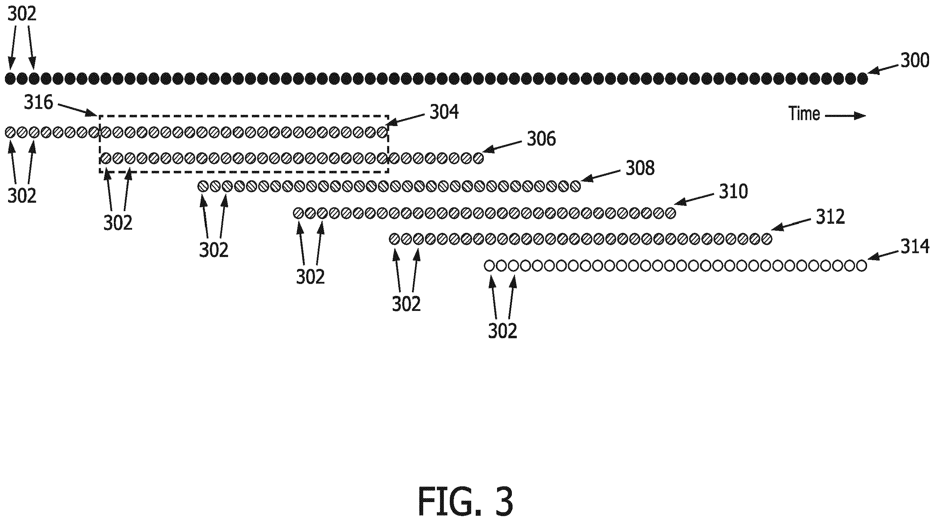

The Doppler processor 160 may extract overlapping time segments of the data for spectral processing. Fig. 3 illustrates overlapping time segments according to principles of the present disclosure. The top line 300 illustrates all of the time samples 302 of the signals acquired at the PRF over time. Each time sample 302 includes data acquired from the entire ROI received from one transmit pulse. The lower lines 304-314 represent overlapping time segments each including a subset of the time samples 302. At least some of the time samples 302 of a time segment (e.g., time segment 304) are the same as at least some of the time samples 302 of a temporally adjacent time segment (e.g., time segment 306) as indicated, for example, by box 316. In some examples, a time segment 304-314 may have a temporal length of approximately 20-40 milliseconds. In some examples, all of the time segments 304-314 may have a same temporal length.

For each time segment 304-314 of data extracted from the clutter-filtered continual stream of data, the 3D spatial location of the vessel (e.g., coronary artery) in the ROI may be determined. For example, the vessel may be detected with R0 and R1 autocorrelation techniques, as if it was a single color flow packet but with an order of magnitude larger packet size. In some examples, projections along each of the spatial dimensions (e.g., azimuth and elevational in addition to axial) may be performed to improve signal to noise ratio or reduce speckle variance in the R0, R1 autocorrelation data.

In some examples, power Doppler data may be used to detect the vessel within the ROI. Power Doppler may be sensitive to the presence of flow, even if does not provide velocity information. In some examples, thresholding of the power Doppler data may be used to detect the vessel. For example, voxels associated with power Doppler values below a threshold value may be discarded and the remaining voxels may be determined to be included in the vessel. In some examples, color flow Doppler data may be used to detect the vessel within the ROI. Color flow Doppler data, while less sensitive to the presence of flow, may provide velocity data. Thresholding techniques may also be used to detect the vessel with color flow Doppler data. For example, voxels associated with velocity magnitudes below a threshold value may be discarded and the remaining voxels may be determined to be included in the vessel.

In some examples, the voxels where flow is detected may be analyzed by the Doppler processor 160 and/or the image processor 136 to determine if the voxels have a vessel-like (e.g., tubular) structure. Relatively broad regions of flow may be discarded/ignored as these may indicate the heart chamber or larger vessel flow. If the vessel in the ROI (e.g., the coronary artery) is not detected, the user interface 124 may alert the user to adjust a location of the ROI and/or the ultrasound probe. Alternatively, a location of the vessel determined from a previous time segment is maintained or tissue motion tracking may be used to translate and rotate the ROI and/or 1D contour from the previous location.

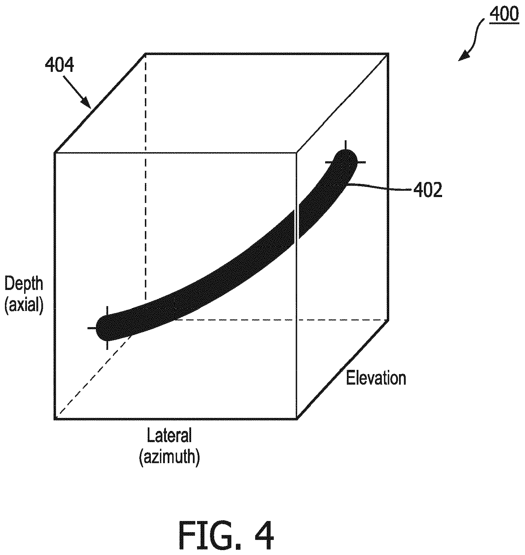

A 1D contour in the 3D ROI may be fitted to the vessel detected by the R0, R1 autocorrelation data vessel (e.g., the voxels determined to be associated with the vessel). The 1D contour may correspond to an M-line along an axis of the vessel in some examples. The contour may include one or more straight line segments or one or more low-order polynomial curves. In some examples, the contour may be fitted to a central axis of the detected vessel voxels. In some examples, the contour may be fitted to voxels having local maximum velocity intensities through the 3D ROI. Fig. 4 is an image of a contour fitted to a vessel according to principles of the present disclosure. Optionally, as illustrated in Fig. 4, a graphic 400 of the contour 402 within the ROI 404 may be generated, for example, by image processor 136 and/or graphics processor 140. In some examples, the graphic 400 may be provided on display 138. In some examples, the graphic may include color flow velocity or power rendering of the vessel derived from the R0, R1 autocorrelations. In some examples, if a graphic showing the vessel position within the ROI on the display 138 also displays color flow velocity or power derived from R1,R0, then the graphic may include a line or other indication to show the maintained or motion tracked 1D contour during a part of the heart cycle when there is no coronary flow.

Spectral PW Doppler data from the overlapping time segments (e.g., time segments 304-314) may be acquired and used to derive velocity estimates, which may be used to generate a spectrogram, for example, by Doppler processor 160 and/or image processor 136, which may be provided on display 138. The spectral PW Doppler data may be coherently integrated along the contour (e.g., contour 402). The coherent integration may be performed for each of the time samples (e.g., time samples 302), not over each of the overlapping time segments. This may improve the spectral spread and signal to noise ratio (SNR) of spectral processing of the PW Doppler data. The different points along the contour may have different axial positions from multiple receive beams. Complex data may be phase and/or time compensated for relative axial position prior to coherent integration along the 1D contour. The coherently integrated data may undergo further spectral processing (e.g., FFT with a tapered window function), for example, in in a traditional periodogram method to generate the spectrogram.

In some examples, separate vessel detection and contour fitting may be performed for individual time segments (e.g., time segments 304-314). Since successive spectral processing batches for generating the spectral PW Doppler data for the spectrogram use potentially slightly different spatial locations for the vessel, vessel translation and/or rotation due to cardiac or other tissue motion or slight transducer movement may be automatically tracked (e.g., the vessel is detected in the individual time segments using the R0, R1 autocorrelation techniques). However, due, at least in part, to the short temporal lengths of the individual time segments, within an individual time segment, the vessel location is fixed for the spectral processing batch. Thus, no spectral artifacts are generated by the vessel tracking. Additionally, the spectral processing batches overlap in time, so the tracking may be smooth. However, in some applications, there may be discontinuities when the vessel is initially detected after a period of no detection.

Furthermore, since the vessel is automatically tracked, the average beam-to-flow angle may be derived. Alternatively, a separate beam-to-contour angle may be calculated for each 1D segment based on a local tangent of the beam to the 1D contour. Thus, the Doppler spectrum provided in the spectrogram may be automatically scaled accordingly to represent true blood velocity, not the projection of that velocity along the transmit/receive beam.

In addition to visualizing the spectral data from the 1D contour as a spectrogram, sonographers and other ultrasound users may find audio data generated from the Doppler data useful for qualitative assessment. As a result, audio output may be optionally produced from the spectral data from the 1D contour. The audio output may be provided on a speaker, such as speaker 170.

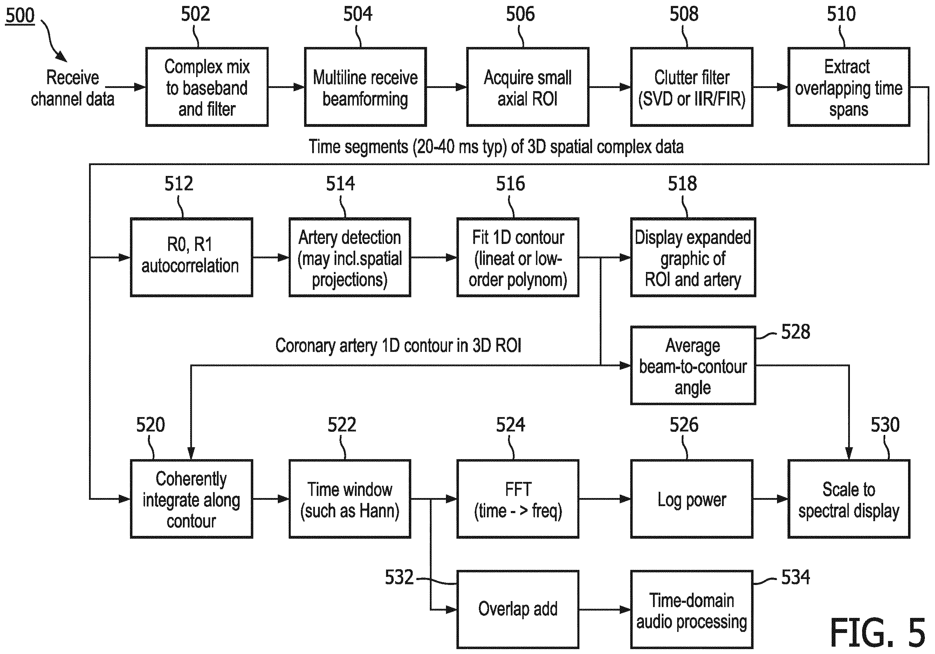

Fig. 5 is a flow chart of a method according to principles of the present disclosure. The method 500 may be performed by an ultrasound imaging system, such as ultrasound imaging system 100. In some examples, the method 500 may be performed, at least in part, by one or more processors, such as signal processor 172, signal processor 126, Doppler processor 160 and/or image processor 136.

In method 500, a transducer array, such as transducer array 114, may transmit electrical signals corresponding to ultrasound data along one or more channels. In some examples, the channels may be conductive structures that transmit signals corresponding to one or more transducer elements of the transducer array. The ultrasound data may correspond to echoes received by the transducer array responsive to ultrasound signals transmitted by the ultrasound array. The transmission and reception of the ultrasound data may be optimized for the acquisition of Doppler data. For example, multiple pulses may be transmitted at a sufficient frequency (e.g., pulse repetition frequency, PRF) to acquire velocity data of flow in a scanned volume (e.g., blood flow velocities within coronary arteries).

As indicated by block 502, a processor, such as signal processor 172, may process the ultrasound data by performing baseband conversion and/or other filtering. A beamformer, such as beamformer 122 may perform beamforming on the processed data as indicated by block 504. In some examples, multiline receive beamforming may be performed, for example, as described with reference to Fig. 2. A user interface, such as user interface 124, may receive a user input indicating a ROI as noted in block 506. The ROI may be a smaller volume (e.g., sub-volume) of a larger volume scanned by the ultrasound probe. In some examples, the ROI may be indicated in the axial (e.g., depth) direction. In some examples, the ROI may be selected by a user from a B-mode image provided on a display, such as display 138.

A signal processor, such as signal processor 126, may process the beamformed data corresponding to data within the ROI as indicated by block 508. The processing may include clutter highpass filtering such as IIR, FIR, and/or other more sophisticated clutter/flow separation techniques filtering. In some examples, the filtered data may be provided to a Doppler processor, such as Doppler processor 160. The filtered data may be a temporal series of data samples. Individual data samples may correspond to different pulses transmitted by the transducer array. In some examples, a temporal spacing between the time samples may correspond to the PRF. As indicated by block 510, the Doppler processor may organize the data samples into one or more overlapping time spans, for example, as described with reference to Fig. 3.

For individual time segments, the Doppler processor may perform R0, R1 autocorrelation techniques as indicated by block 512 to extract various types of Doppler data (e.g., color flow, power). The Doppler processor may use the autocorrelation data to detect a vessel within the ROI. For example, the Doppler data (e.g., power, flow, velocity) may be compared to a threshold value to determine if the voxel corresponding to the Doppler data is within a vessel. In some examples, the Doppler processor may analyze the voxels indicated as including flow to determine if the voxels form a vessel-like structure.

As indicated by block 516, the Doppler processor, or an image processor, such as image processor 136, may fit a 1D contour to the detected vessel. In some examples, the image processor and/or a graphics processor, such as graphics processor 140, may generate display information for displaying the 1D contour on the display, as noted by block 518.

The Doppler processor may integrate spectral PW Doppler data of individual time segments along the 1D contour corresponding to the vessel as indicated by 520. In some examples, the integrating may be coherent integration. Integrating along the contour is in contrast to conventional spectral PW Doppler which only analyzes data in a short-range gate along a single axial line. However, similar to conventional spectral PW Doppler, the Doppler processor may apply time windowing (e.g., a Hann window) to the integrated data and applies a FFT to extract the Doppler frequencies from the spectral PW Doppler as indicated by blocks 522 and 524.

As indicated by block 526, the Doppler processor 160 may take a log power of the Doppler frequency data, also similar to conventional spectral PW Doppler data processing. However, the 1D contour calculated at block 516 may also be used by the Doppler processor to calculate the average ultrasound beam-to-contour angle as indicated by block 528. This may be used to correct the log power data to provide corrected velocities. Alternatively, an FFT may be performed for individual portions of the 1D contour, and a separate beam-to-contour angle may be calculated for each 1D segment based on a local tangent of the beam to the 1D contour. The complex spectra, (which may each have a different frequency interpolation) may be summed prior to taking the log power.

As indicated by block 520, the velocities derived from the spectral PW Doppler data may be plotted as a spectrogram and provided on the display as indicated by block 530. The display information for the spectrogram may be generated by the image processor and/or the graphics processor based on the Doppler data provided by the Doppler processor.

Audio output may be optionally produced from the spectral PW Doppler data generated by the Doppler processor. The Doppler processor may produce an audio signal from the overlapping time segments of data integrated along the contour of the detected vessel. In some examples, as indicated by block 532, the Doppler processor may perform an overlap-add method reconstructing a continual stream of data. The Doppler processor may then perform time-domain audio processing on the data from the time segments to generate the audio output as indicated by block 534. However, in other examples, the audio output may be constructed in the frequency domain after the spectral FFT. The audio output may be provided on a speaker, such as speaker 170.

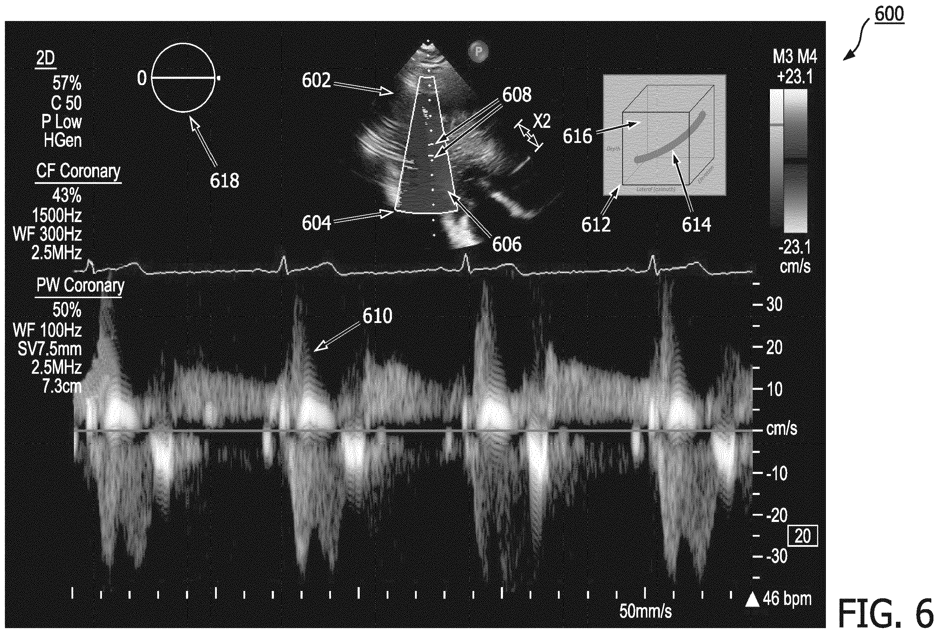

Fig. 6 is an example of an output of a display of an ultrasound imaging system in accordance with principles of the present disclosure. In some examples, the output 600 may be provided on display 138 of ultrasound imaging system 100.

The output 600 may include a B-mode image 602. The B-mode image 602 may have been acquired by an ultrasound probe, such as ultrasound probe 112. The output 600 may further include an indication of a ROI 604. The ROI 604 may indicate a region for Doppler color flow imaging. In the example shown in Fig. 6, a color flow image 606 is overlaid on the B-mode image 602. Within ROI 604, two lines indicate an ROI 608 where spectral PW Doppler data is desired. In some examples, ROI 604 and/or 608 may be indicated by a user input received by a user interface, such as user interface 124. In other examples, ROI 604 and/or ROI 608 may be automatically selected by the ultrasound imaging system (e.g., based on detection of flow or image segmentation techniques used for recognizing vessels). In some examples, at least a portion of a vessel, such as a coronary artery, may be located within ROI 608.

A spectrogram 610 indicating the velocities present in the vessel located in ROI 608 may also be provided in output 600. In some examples, the spectrogram 610 may have been generated by the techniques disclosed herein, such as those described with reference to Figs. 1-5. In some examples, the output 600 may further include a graphic 612 of a 1D contour of the portion of the vessel located within ROI 608. While acquiring the spectral PW Doppler data, the user must maintain the transducer position. With conventional uninterrupted PW Doppler, this is challenging, particularly for coronary arteries because there is no visual feedback besides the spectrogram, and the vessel may be difficult to find with color flow even before entering the spectral imaging mode. The spatial tracking described herein may automatically track small movement of the vessel (which may be primarily due to cardiac motion in some applications), but the user must still maintain the transducer position well enough to keep the coronary artery within the ROI indicated by 608 in some examples, unless the system automatically moves the transmit and ROI position.

Maintaining transducer position may be aided by displaying the real-time (or near real-time) graphic 612 showing the detected vessel 614 within the outlines of a 3D ROI 616, which may correspond to ROI 608. The vessel 614 may be displayed either as a graphic line (as shown in Figs. 4 and 6) or as a 3D color flow image using R0, R1 data. The spectral PW Doppler acquisition and spectrogram are not interrupted to produce the graphic 612, because the graphic 612 is derived from the multiline PW Doppler data. However, in other examples, additional tracking techniques may be used to track gross movements of the vessel (e.g., due to probe motion) and the location of ROI 608 may automatically be adjusted responsive to the tracking.

In some examples, the output 600 may further include an indication of the Doppler angle 618. The indication of the Doppler angle 618 may allow the user to adjust the orientation of the probe to maintain the beam as close as possible to parallel with the flow within the vessel as velocity cannot be measured when the beam is perpendicular to the direction of flow. Even when the Doppler angle is corrected for in the calculation of velocities, accuracy is improved when the Doppler angle is reduced. As shown in Fig. 6, the output 600 may allow several elements, such as the B-mode image 602, the color flow image 606, and the graphic 612 to be displayed simultaneously.

The systems and methods disclosed herein may provide techniques for automatically finding and tracking a vessel, such as a coronary artery, in a small spatial region, and coherently enhancing spectral PW Doppler ultrasound for quantifying its blood flow, including automatic angle correction and audio output. A live 3D graphic or color flow image may be generated, which may help a user maintain transducer position during uninterrupted spectrogram acquisition and display. Although the examples provided herein refer to coronary arteries, the systems and methods disclosed herein may also be used with other anatomy.

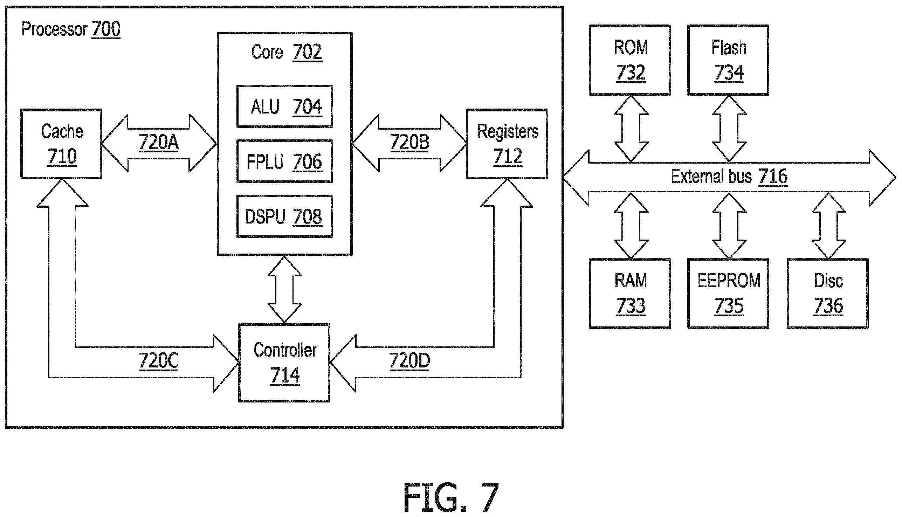

Fig. 7 is a block diagram illustrating an example processor 700 according to principles of the present disclosure. Processor 700 may be used to implement one or more processors described herein, for example, image processor 136 and/or Doppler processor 160 shown in Fig. 1. Processor 700 may be capable of executing computer-readable instructions stored on a non-transitory computer-readable medium in communication with the processor 700, for example, local memory 142 shown in Fig. 1. Processor 700 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 700 may include one or more cores 702. The core 702 may include one or more arithmetic logic units (ALU) 704. In some examples, the core 702 may include a floating-point logic unit (FPLU) 706 and/or a digital signal processing unit (DSPU) 708 in addition to or instead of the ALU 704.

The processor 700 may include one or more registers 712 communicatively coupled to the core 702. The registers 712 may be implemented using dedicated logic gate circuits (e.g., flip-flops) and/or any memory technology. In some examples the registers 712 may be implemented using static memory. The register may provide data, instructions and addresses to the core 702.

In some examples, processor 700 may include one or more levels of cache memory 710 communicatively coupled to the core 702. The cache memory 710 may provide computer-readable instructions to the core 702 for execution. The cache memory 710 may provide data for processing by the core 702. In some examples, the computer-readable instructions may have been provided to the cache memory 710 by a local memory, for example, local memory attached to the external bus 716. The cache memory 710 may be implemented with any suitable cache memory type, for example, metal-oxide semiconductor (MOS) memory such as static random-access memory (SRAM), dynamic random-access memory (DRAM), and/or any other suitable memory technology.

The processor 700 may include a controller 714, which may control input to the processor 700 from other processors and/or components included in a system (e.g., control panel 152 and scan converter 130 shown in Fig. 1) and/or outputs from the processor 700 to other processors and/or components included in the system (e.g., display 138 and volume renderer 134 shown in Fig. 1). Controller 714 may control the data paths in the ALU 704, FPLU 706 and/or DSPU 708. Controller 714 may be implemented as one or more state machines, data paths and/or dedicated control logic. The gates of controller 714 may be implemented as standalone gates, FPGA, ASIC or any other suitable technology.

The registers 712 and the cache memory 710 may communicate with controller 714 and core 702 via internal connections 720A, 720B, 720C and 720D. Internal connections may be implemented as a bus, multiplexor, crossbar switch, and/or any other suitable connection technology.

Inputs and outputs for the processor 700 may be provided via a bus 716, which may include one or more conductive lines. The bus 716 may be communicatively coupled to one or more components of processor 700, for example the controller 714, cache memory 710, and/or register 712. The bus 716 may be coupled to one or more components of the system, such as display 138 and control panel 152 mentioned previously.

The bus 716 may be coupled to one or more external memories. The external memories may include Read Only Memory (ROM) 732. ROM 732 may be a masked ROM, Electronically Programmable Read Only Memory (EPROM) or any other suitable technology. The external memory may include Random Access Memory (RAM) 733. RAM 733 may be a static RAM, battery backed up static RAM, Dynamic RAM (DRAM) or any other suitable technology. The external memory may include Electrically Erasable Programmable Read Only Memory (EEPROM) 735. The external memory may include Flash memory 734. The external memory may include a magnetic storage device such as disc 736. In some examples, the external memories may be included in a system, such as ultrasound imaging system 100 shown in Fig. 1, for example local memory 142.

In various examples where components, systems and/or methods are implemented using a programmable device, such as a computer-based system or programmable logic, it should be appreciated that the above-described systems and methods can be implemented using any of various known or later developed programming languages, such as "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure, it is noted that the various methods and devices described herein can be implemented in hardware, software, and/or firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general-purpose processing circuits which are programmed responsive to executable instructions to perform the functions described herein.

The present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, examples or processes described herein may be combined with one or more other examples, examples and/or processes or be separated and/or performed amongst separate devices or device portions in accordance with the present systems, devices and methods.

Finally, the above discussion is intended to be merely illustrative of the present systems and methods and should not be construed as limiting the appended claims to any particular example or group of examples. Thus, while the present system has been described in particular detail with reference to exemplary examples, it should also be appreciated that numerous modifications and alternative examples may be devised by those having ordinary skill in the art without departing from the broader and intended scope of the present systems and methods as set forth in the claims that follow. Accordingly, the specification and drawings are to be regarded in an illustrative manner and are not intended to limit the scope of the appended claims.

## Claims

1. A computer-implemented method for providing a spectrogram of blood flow velocities from ultrasound data, the method comprising:
performing, with a beamformer (122), multi-line receive beamforming on ultrasound data;
generating, with at least one processor (160, 136, 140), image data from the beamformed ultrasound data;
organizing, with at least one processor (160, 136, 140), the image data received from the beamformer into a plurality of overlapping time segments;
autocorrelating individual ones of the plurality of overlapping time segments;
detecting a vessel in the image data of individual ones of the plurality of overlapping time segments based, at least in part, on the autocorrelating;
fitting a one-dimensional (1D) contour to the vessel;
integrating the image data from at least a portion of the 1D contour for individual time samples of the individual ones of the plurality of overlapping time segments;
extracting a plurality of Doppler frequencies from the integrated image data of the individual ones of the plurality of overlapping time segments;
calculating an angle between an ultrasound beam used to acquire the image data and the 1D contour;
converting the plurality of Doppler frequencies to a plurality of velocities based, at least in part, on the angle; and
plotting the plurality of velocities for individual ones of the plurality of overlapping time segments on a spectrogram.

2. The computer-implemented method of claim 1, further comprising acquiring the ultrasound data by transmitting a plurality of ultrasound pulses with a transducer array (114), wherein a temporal spacing of the plurality of ultrasound pulses is based, at least in part, on velocities of flow in the vessel.

3. The computer-implemented method of claim 1, further comprising receiving a user input from a user interface (124), wherein the user input indicates a region of interest. and wherein ultrasound data outside the region of interest is not provided to the at least one processor.

4. The computer-implemented method of claim 1, further comprising clutter filtering the ultrasound data prior to the organizing of the ultrasound data into the plurality of overlapping time segments.

5. The computer-implemented method of claim 1, further comprising performing a baseband conversion on the ultrasound data prior to beamforming.

6. The computer-implemented method of claim 1, further comprising performing time domain processing on the plurality of Doppler frequencies.

7. The computer-implemented method of claim 1, further comprising generating an audio output based, at least in part, on the plurality of velocities.

8. The computer-implemented method of claim 1, further comprising applying a time window to the image data after the integrating and prior to extracting the plurality of Doppler frequencies.

9. The computer-implemented method of claim 8, wherein the time window comprises a Hann window.

10. The computer-implemented method of claim 1, wherein converting the plurality of Doppler frequencies to the plurality of velocities is further based on a log power of the plurality of Doppler frequencies.

11. The computer-implemented method of claim 1, further comprising providing a graphic of the 1D contour.

12. The computer-implemented method of claim 11, further comprising providing a line on the graphic indicating a maintained or a motion tracking of the 1D contour during a part of a heart cycle when there is no coronary flow.

13. The computer-implemented method of claim 11, further comprising providing a B-mode image (602) and a color flow Doppler image (606) simultaneously with the 1D contour.

14. The computer-implemented method of claim 1, further comprising:
transmitting ultrasound beams and receiving the ultrasound data responsive to the ultrasound beams; and
steering the ultrasound beams based, at least in part, on the 1D contour.

15. An ultrasound imaging system for providing a spectrogram of blood flow velocities from ultrasound data, the system comprising a beamformer (122) and at least one processor (160, 136, 140), the system being configured to carry out the method of any of claims 1 to 14.

16. A computer program product comprising instructions for enabling a processor to carry out the method of any of claims 1 to 14.

## Patentansprüche

1. Computerimplementiertes Verfahren zum Bereitstellen eines Spektrogramms der Blutflussgeschwindigkeiten aus Ultraschalldaten, wobei das Verfahren umfasst:
Durchführen, mit einem Strahlbildner (122), einer mehrzeiligen Empfangs-Strahlbildung an Ultraschalldaten;
Erzeugen, mit mindestens einem Prozessor (160, 136, 140), von Bilddaten aus den strahlgebildeten Ultraschalldaten;
Organisieren, mit mindestens einem Prozessor (160, 136, 140), der vom Strahlbildner empfangenen Bilddaten in eine Vielzahl von überlappenden Zeitsegmenten;
Autokorrelieren einzelner der Vielzahl von überlappenden Zeitsegmenten;
Erkennen eines Gefäßes in den Bilddaten einzelner der Vielzahl von überlappenden Zeitsegmenten, basierend zumindest teilweise auf der Autokorrelation;
Anpassen einer eindimensionalen (1D) Kontur an das Gefäß;
Integrieren der Bilddaten von zumindest einem Abschnitt der 1D-Kontur für einzelne Zeitproben der einzelnen der Vielzahl von überlappenden Zeitsegmenten;
Extrahieren einer Vielzahl von Dopplerfrequenzen aus den integrierten Bilddaten der einzelnen der Vielzahl von überlappenden Zeitsegmenten;
Berechnen eines Winkels zwischen einem Ultraschallstrahl, der verwendet wird, um die Bilddaten zu erfassen, und der 1D-Kontur;
Umwandeln der Vielzahl von Dopplerfrequenzen in eine Vielzahl von Geschwindigkeiten zumindest teilweise auf dem Winkel basierend; und
Aufzeichnen der Vielzahl von Geschwindigkeiten für einzelne der Vielzahl von überlappenden Zeitsegmenten in einem Spektrogramm.

2. Computerimplementiertes Verfahren nach Anspruch 1, das weiter das Erfassen der Ultraschalldaten durch Senden einer Vielzahl von Ultraschallimpulsen mit einem Wandlerarray (114) umfasst, wobei ein zeitlicher Abstand der Vielzahl von Ultraschallimpulsen zumindest teilweise auf Strömungsgeschwindigkeiten im Gefäß basiert.

3. Computerimplementiertes Verfahren nach Anspruch 1, das weiter das Empfangen einer Benutzereingabe von einer Benutzerschnittstelle (124) umfasst, wobei die Benutzereingabe einen Bereich von Interesse angibt und wobei Ultraschalldaten außerhalb des Bereichs von Interesse dem mindestens einen Prozessor nicht bereitgestellt werden.

4. Computerimplementiertes Verfahren nach Anspruch 1, das weiter das Clutter-Filtern der Ultraschalldaten vor dem Organisieren der Ultraschalldaten in die Vielzahl von überlappenden Zeitsegmenten umfasst.

5. Computerimplementiertes Verfahren nach Anspruch 1, das weiter das Durchführen einer Basisbandkonvertierung der Ultraschalldaten vor der Strahlbildung umfasst.

6. Computerimplementiertes Verfahren nach Anspruch 1, das weiter das Durchführen einer Zeitdomänen-Verarbeitung der Vielzahl von Dopplerfrequenzen umfasst.

7. Computerimplementiertes Verfahren nach Anspruch 1, das weiter das Erzeugen einer Audioausgabe umfasst, die zumindest teilweise auf der Vielzahl von Geschwindigkeiten basiert.

8. Computerimplementiertes Verfahren nach Anspruch 1, das weiter das Anwenden eines Zeitfensters auf die Bilddaten nach dem Integrieren und vor dem Extrahieren der Vielzahl von Dopplerfrequenzen umfasst.

9. Computerimplementiertes Verfahren nach Anspruch 8, wobei das Zeitfenster ein Hann-Fenster umfasst.

10. Computerimplementiertes Verfahren nach Anspruch 1, wobei das Umwandeln der Vielzahl von Dopplerfrequenzen in die Vielzahl von Geschwindigkeiten weiter auf einer Log-Leistung der Vielzahl von Dopplerfrequenzen basiert.

11. Computerimplementiertes Verfahren nach Anspruch 1, das weiter das Bereitstellen einer Grafik der 1D-Kontur umfasst.

12. Computerimplementiertes Verfahren nach Anspruch 11, das weiter das Bereitstellen einer Linie in der Grafik umfasst, die eine Beibehaltung oder Bewegungsverfolgung der 1D-Kontur während eines Teils eines Herzzyklus angibt, wenn kein Koronarfluss vorhanden ist.

13. Computerimplementiertes Verfahren nach Anspruch 11, das weiter das Bereitstellen eines B-Mode-Bildes (602) und eines Farbfluss-Dopplerbildes (606) gleichzeitig mit der 1D-Kontur umfasst.

14. Computerimplementiertes Verfahren nach Anspruch 1, weiter umfassend:
Senden von Ultraschallstrahlen und Empfangen der Ultraschalldaten als Reaktion auf die Ultraschallstrahlen; und
Lenken der Ultraschallstrahlen zumindest teilweise basierend auf der 1D-Kontur.

15. Ultraschallbildgebungssystem zum Bereitstellen eines Spektrogramms der Blutflussgeschwindigkeiten aus Ultraschalldaten, wobei das System einen Strahlbildner (122) und mindestens einen Prozessor (160, 136, 140) umfasst, wobei das System konfiguriert ist, um das Verfahren nach einem der Ansprüche 1 bis 14 auszuführen.

16. Computerprogrammprodukt, das Computeranweisungen zum Aktivieren eines Prozessors umfasst, das Verfahren nach einem der Ansprüche 1 bis 14 auszuführen.

## Revendications

1. Procédé mis en oeuvre par ordinateur pour fournir un spectrogramme de vitesses d'écoulement sanguin à partir de données ultrasonores, le procédé comprenant les étapes consistant à :
effectuer, avec un formateur de faisceau (122), une formation de faisceau de réception multiligne sur des données ultrasonores ;
générer, avec au moins un processeur (160, 136, 140), des données d'image à partir des données ultrasonores formées en faisceau ;
organiser, avec au moins un processeur (160, 136, 140), les données d'image reçues à partir du formateur de faisceau en une pluralité de segments temporels se chevauchant ;
autocorréler des segments temporels individuels de la pluralité de segments temporels se chevauchant ;
détecter un vaisseau dans les données d'image de segments temporels individuels de la pluralité de segments temporels se chevauchant sur la base, au moins en partie, de l'autocorrélation ;
ajuster un contour unidimensionnel (1D) au vaisseau ;
intégrer les données d'image à partir d'au moins une partie du contour 1D pour des échantillons temporels individuels des segments temporels individuels de la pluralité de segments temporels se chevauchant ;
extraire une pluralité de fréquences Doppler à partir des données d'image intégrées des segments temporels individuels de la pluralité de segments temporels se chevauchant ;
calculer un angle entre un faisceau ultrasonore utilisé pour acquérir les données d'image et le contour 1D ;
convertir la pluralité de fréquences Doppler en une pluralité de vitesses sur la base, au moins en partie, de l'angle ; et
tracer la pluralité de vitesses pour des segments temporels individuels de la pluralité de segments temporels se chevauchant sur un spectrogramme.

2. Procédé mis en oeuvre par ordinateur selon la revendication 1, comprenant en outre une acquisition des données ultrasonores en transmettant une pluralité d'impulsions ultrasonores avec un réseau de transducteurs (114), dans lequel un espacement temporel de la pluralité d'impulsions ultrasonores est basé, au moins en partie, sur des vitesses d'écoulement dans le vaisseau.

3. Procédé mis en oeuvre par ordinateur selon la revendication 1, comprenant en outre une réception d'une entrée utilisateur à partir d'une interface utilisateur (124), dans lequel l'entrée utilisateur indique une région d'intérêt, et dans lequel des données ultrasonores en dehors de la région d'intérêt ne sont pas fournies au au moins un processeur.

4. Procédé mis en oeuvre par ordinateur selon la revendication 1, comprenant en outre un filtrage de fouillis des données ultrasonores avant l'organisation des données ultrasonores dans la pluralité de segments temporels se chevauchant.

5. Procédé mis en oeuvre par ordinateur selon la revendication 1, comprenant en outre une réalisation d'une conversion en bande de base sur les données ultrasonores avant la formation de faisceau.

6. Procédé mis en oeuvre par ordinateur selon la revendication 1, comprenant en outre une réalisation d'un traitement de domaine temporel sur la pluralité de fréquences Doppler.

7. Procédé mis en oeuvre par ordinateur selon la revendication 1, comprenant en outre une génération d'une sortie audio sur la base, au moins en partie, de la pluralité de vitesses.

8. Procédé mis en oeuvre par ordinateur selon la revendication 1, comprenant en outre une application d'une fenêtre temporelle aux données d'image après l'intégration et avant l'extraction de la pluralité de fréquences Doppler.

9. Procédé mis en oeuvre par ordinateur selon la revendication 8, dans lequel la fenêtre temporelle comprend une fenêtre de Hann.

10. Procédé mis en oeuvre par ordinateur selon la revendication 1, dans lequel la conversion de la pluralité de fréquences Doppler en la pluralité de vitesses est en outre basée sur une puissance logarithmique de la pluralité de fréquences Doppler.

11. Procédé mis en oeuvre par ordinateur selon la revendication 1, comprenant en outre une fourniture d'un graphique du contour 1D.

12. Procédé mis en oeuvre par ordinateur selon la revendication 11, comprenant en outre une fourniture d'une ligne sur le graphique indiquant un contour 1D maintenu ou un suivi de mouvement du contour 1D pendant une partie d'un cycle cardiaque lorsqu'il n'y a pas d'écoulement coronaire.

13. Procédé mis en oeuvre par ordinateur selon la revendication 11, comprenant en outre une fourniture d'une image en mode B (602) et d'une image Doppler d'écoulement couleur (606) simultanément avec le contour 1D.

14. Procédé mis en oeuvre par ordinateur selon la revendication 1, comprenant en outre les étapes consistant à :
transmettre des faisceaux ultrasonores et recevoir les données ultrasonores en réponse aux faisceaux ultrasonores ; et
orienter les faisceaux ultrasonores sur la base, au moins en partie, du contour 1D.

15. Système d'imagerie ultrasonore pour fournir un spectrogramme de vitesses d'écoulement sanguin à partir de données ultrasonores, le système comprenant un formateur de faisceau (122) et au moins un processeur (160, 136, 140), le système étant configuré pour effectuer le procédé selon l'une quelconque des revendications 1 à 14.

16. Produit-programme informatique comprenant des instructions pour permettre à un processeur d'effectuer le procédé selon l'une quelconque des revendications 1 à 14.
